(19) Europäisches Patentamt
European Patent Office
Office européen des brevets

(11) EP 1 285 947 B1

(12) **EUROPEAN PATENT SPECIFICATION**

(45) Date of publication and mention of the grant of the patent:
**20.02.2008 Bulletin 2008/08**

(21) Application number: **01930196.9**

(22) Date of filing: **17.05.2001**

(51) Int Cl.:
***A61K 31/4425*** *(2006.01)* ***A61K 6/00*** *(2006.01)*

(86) International application number:
**PCT/JP2001/004134**

(87) International publication number:
**WO 2001/090251 (29.11.2001 Gazette 2001/48)**

(54) **ANTIMICROBIAL COMPOSITION**

ANTIMIKROBIELLE ZUSAMMENSETZUNG

COMPOSITION ANTIMICROBIENNE

(84) Designated Contracting States:
**AT BE CH CY DE DK ES FI FR GB GR IE IT LI LU MC NL PT SE TR**

(30) Priority: **22.05.2000 JP 2000149913**

(43) Date of publication of application:
**26.02.2003 Bulletin 2003/09**

(73) Proprietor: **KURARAY CO., LTD.**
**Kurashiki-shi, Okayama 710-8622 (JP)**

(72) Inventor: **NAKATSUKA, Kazumitsu**
**Kurashiki-shi**
**Okayama 710-8622 (JP)**

(74) Representative: **Müller-Boré & Partner**
**Patentanwälte**
**Grafinger Strasse 2**
**81671 München (DE)**

(56) References cited:
**EP-A1- 0 705 590**
**WO-A-99/51230**
**JP-A- 7 082 115**
**JP-A- 7 138 119**
**JP-A- 2000 063 222**

- **PATENT ABSTRACTS OF JAPAN vol. 012, no. 165 (C-496), 18 May 1988 (1988-05-18) & JP 62 277323 A (SANKYO CO LTD), 2 December 1987 (1987-12-02)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 285 947 B1

## Description

### TECHNICAL FIELD

The present invention relates to an antibacterial composition. More precisely, the invention relates to an antibacterial composition for dental use for treating decayed teeth, especially to an adhesive antibacterial composition having the ability to kill cariogenic bacteria in decayed teeth or to prevent the bacteria therein from propagating and having the ability to enhance the adhesiveness of dental bonding materials, dental cement materials, composite resins for dental restoration, compomers for dental restoration, etc.

### BACKGROUND ART

In dental treatment where partial defects in teeth are restored through prosthesis with restorative dental materials such as, for example, composite resins, compomers, metal alloys, ceramics and the like for dental restoration, often used are acrylic dental bonding compositions. However, even when such dental bonding compositions are used for that purpose, bacteria may often penetrate into the restored tooth through the bonding interface between the tooth and the bonding composition to thereby cause secondary caries or odontitis. In that case, the restored tooth will need to be retreated. For preventing bacteria from penetrating into restored teeth, bonding compositions having the ability to well seal up the bonded area of the restored tooth are effective. For example, a bonding system of using a so-called self-etching primer that contains an acid group-having polymerizable monomer and a hydrophilic polymerizable monomer is favorably employed in the art, as its ability to bond to teeth and to seal up the bonded teeth is good. In addition to improving the adhesiveness of dental bonding compositions, adding an antibacterial compound to the compositions is tried. For example, JP-A 157318/1996 discloses an antibacterial composition comprising an acid group-having polymerizable monomer, a hydrophilic polymerizable monomer, water and a polymerization initiator and containing a polymerizable group-having antibacterial pyridinium salt. The antibacterial composition disclosed keeps good adhesiveness to the teeth to which it has been applied, and the technique of using it is for killing the cariogenic bacteria that may remain in the restored teeth. The composition disclosed is well effective for killing cariogenic bacteria when the number of the bacteria is small, but is problematic in that it could not satisfactorily exhibit its antibacterial capability when the number of the bacteria increases or when the time of contact between the composition and the bacteria is short.

Another problem with the antibacterial composition disclosed in that patent publication is that it discolors while stored at room temperature for a long period of time, and, in particular, when it is stored at high temperatures, it becomes blackish. Therefore, if the composition is used for treating and restoring teeth after stored at room temperature for a long period of time, the bonded area (and therearound) of the restored teeth will be brownish, and therefore it fails to esthetically treat teeth. In addition to the problem of discoloration, still another problem with the composition is that its adhesiveness to dentin lowers time-dependently while stored long. Therefore, it is desired to further improve the storage stability of the composition.

WO-A-99/51230 describes a method for stabilizing a pharmaceutical composition by contacting said composition with a polymeric material comprising an antioxidant.

The problem that the invention is to solve is how to provide an antibacterial composition having the advantages of more improved antibacteriality and good storage stability in that, even when stored for a long period of time or heated, it discolors little and its adhesiveness lowers little.

On the basis of the antibacterial composition described in JP-A 157318/1996, the present inventor has further assiduously studied to improve the antibacterial property and the storage stability of the composition, and, surprisingly as a result, has found that, when a basic compound selected from alkali metal hydroxides, strong basic acid salts not having an aromatic group, and aliphatic amines is added to an antibacterial composition comprising an antibacterial salt compound, an acid group-having polymerizable monomer, a hydrophilic polymerizable monomer and water, then the object as above can be attained. On the basis of this finding, the inventor has completed the present invention.

### DISCLOSURE OF THE INVENTION

The invention is an antibacterial composition comprising (a) a polymerizable group-having antibacterial salt compound, (b) an acid group-having polymerizable monomer, (c) a hydrophilic polymerizable monomer, (d) water, and (e) a basic compound selected from alkali metal hydroxides, strong basic acid salts not having an aromatic group, and aliphatic amines.

### BRIEF DESCRIPTION OF THE DRAWING

Fig. 1 shows a process of producing antibacterial methacrylic pyridinium salts.

BEST MODES OF CARRYING OUT THE INVENTION

The antibacterial salt compound (a) to be used in the antibacterial composition of the invention is a polymerizable group-having antibacterial salt compound. The antibacterial salt compound includes, for example, various antibacterial ammonium salt compounds of the following general formulae (I).

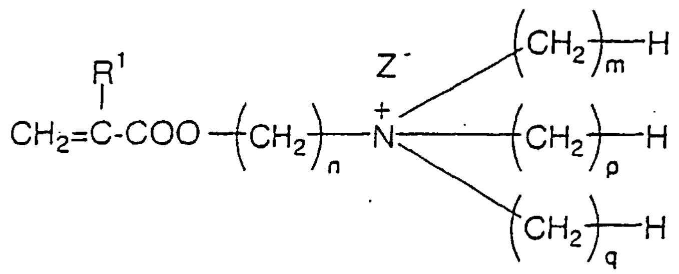

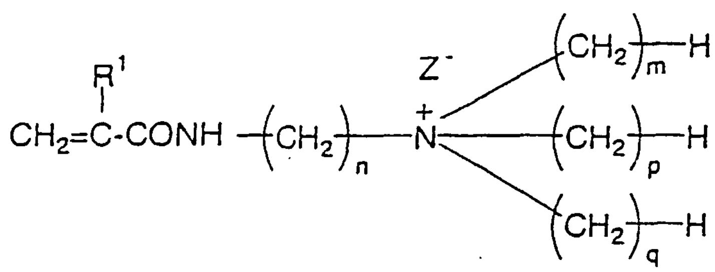

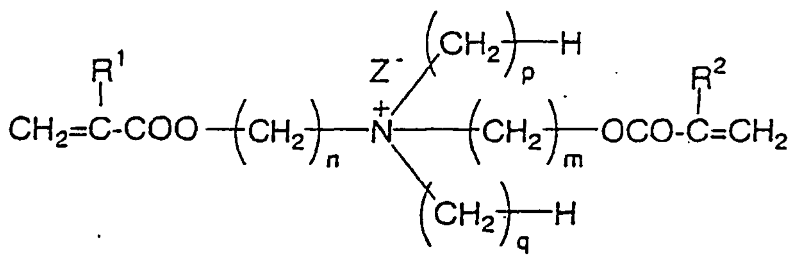

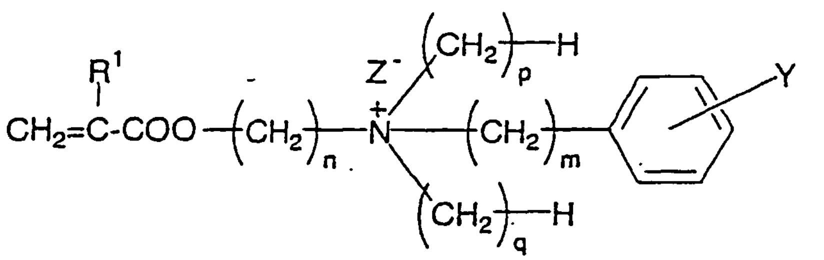

(I)

wherein $R^1$ represents H or $CH_3$; $R^2$ represents H or $CH_3$; Y represents $OCH_3$, $OCH_2CH_3$, $OCH_2CH_2CH_3$, Cl, Br, COOH, OH, CN or $CH=CH_2$; Z represents F, Cl, Br, I, $1/2PO_4$, $1/2SO_4$, $CH_3$-$SO_3$, $CH_3COO$; n represents an integer of from 1

to 30; m represents an integer of from 1 to 30; p represents an integer of from 1 to 30; and q represents an integer of from 1 to 30.

The compound further includes other various antibacterial phosphonium salt compounds of the following general formulae (II).

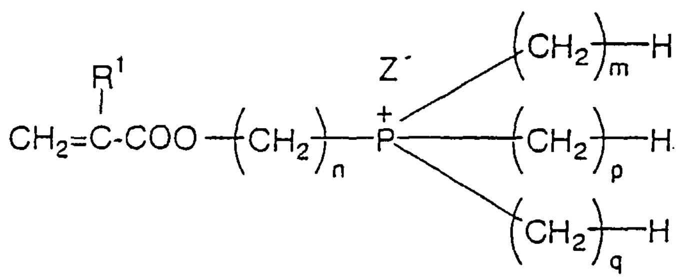

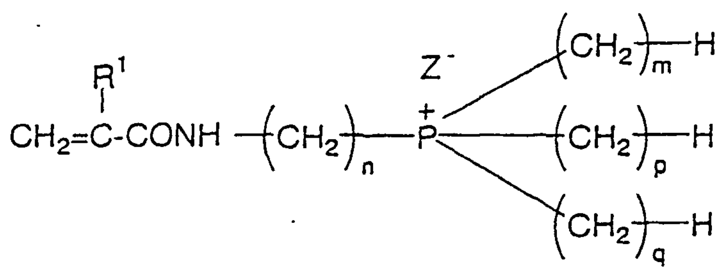

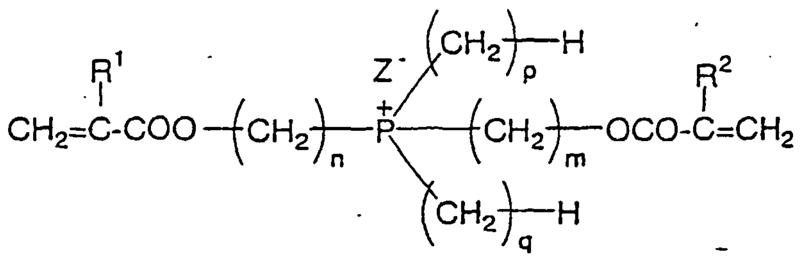

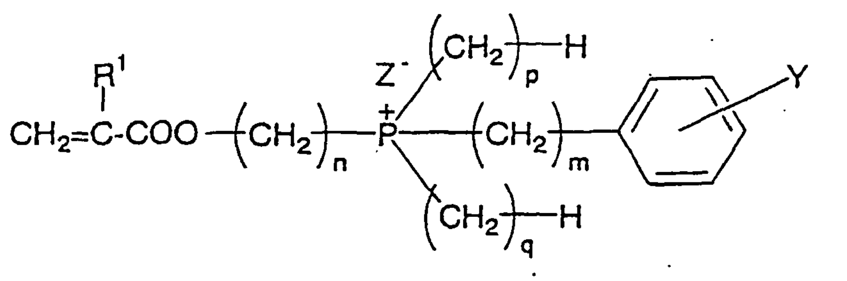

(I I)

wherein $R^1$ represents H or $CH_3$; $R^2$ represents H or $CH_3$; Y represents $OCH_3$, $OCH_2CH_3$, $OCH_2CH_2CH_3$, Cl, Br, COOH, OH, CN or $CH=CH_2$; Z represents F, Cl, Br, I, $1/2PO_4$, $1/2SO_4$, $CH_3$-$SO_3$, $CH_3COO$; n represents an integer of from 1 to 30; m represents an integer of from 1 to 30; p represents an integer of from 1 to 30; and q represents an integer of from 1 to 30.

The compound still further includes other various antibacterial pyridinium salt compounds of the following general formula (III).

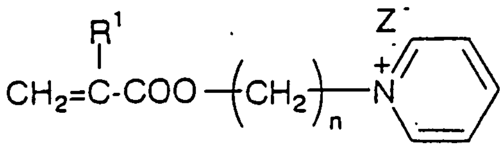

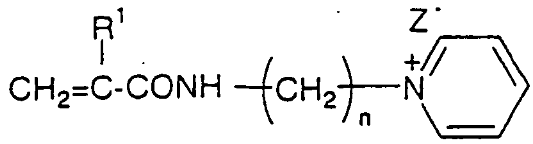

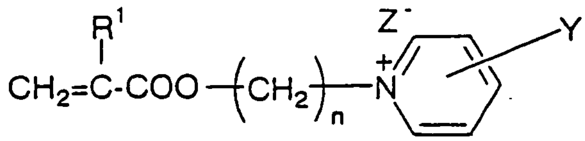

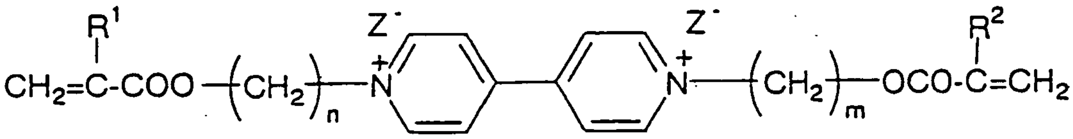

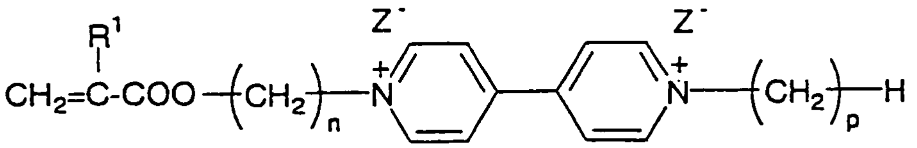

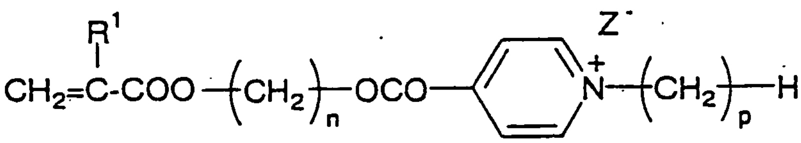

(III)

wherein $R^1$ represents H or $CH_3$; $R^2$ represents H or $CH_3$; Y represents $OCH_3$, $OCH_2CH_3$, $OCH_2CH_2CH_3$, Cl, Br, COOH, OH, or CN; Z represents F, Cl, Br, I, $1/2PO_4$, $1/2SO_4$, $CH_3$-$SO_3$, $CH_3COO$; n represents an integer of from 1 to 30; m

represents an integer of from 1 to 30 ; and p represents an integer of from 1 to 30.

Of those, preferred for use in the invention are compounds having, in the molecule, an alkyl group and an alkylene group with from 10 to 20 carbon atoms, as their antibacterial property is good. Also preferred are antibacterial pyridinium salt compounds, as they exhibit good antibacteriality in the presence of an acid group-having polymerizable monomer. According to the present invention, the antibacterial salt compounds have a polymerizable group in the molecule. Such polymerizable group-having antibacterial salt compounds can be fixed on teeth through polymerization thereon, and after polymerized and cured on teeth, they are effective for preventing the penetration of bacteria into the bonded teeth through the bonding interface. In addition, when compared with other antibacterial salt compounds not having a polymerizable group, the polymerizable group-having antibacterial salt compounds of the type are better, as the antibacterial compositions comprising them ensure better adhesiveness to teeth.

Of the antibacterial salt compounds mentioned above, especially preferred for use herein are antibacterial methacrylic pyridinium salts of the following general formula (IV), such as typically methacryloyloxydodecylpyridinium bromide (hereinafter referred to as MDPB), as they are easy to produce.

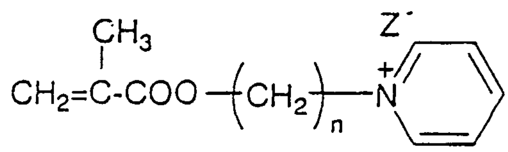

(I V)

wherein Z represents Cl, Br, or I; n represents an integer of from 12 to 25.

One example of the method for producing antibacterial methacrylic pyridinium salts is shown in Fig. 1, to which, however, the production method is not limited. In the process shown in Fig. 1, the reagents for esterification and halogenation may be any known ones, not specifically defined. In this, if desired, the methacrylic acid to be used may be pre-activated, for example, through halogenation or tosylation.

One or more different types of such antibacterial salt compounds may be used herein either singly or as combined. Regarding its blend ratio in the composition, if the amount of the antibacterial salt compound therein is too small, the composition could not be antibacterial; but if too large, the adhesiveness of the composition will lower. Accordingly, the amount of the antibacterial salt compound in the composition may fall generally between 0.01 and 25 % by weight of the composition, but preferably between 0.1 and 15 % by weight, more preferably between 1 and 10 % by weight.

In the invention, the acid group-having polymerizable monomer (b) is indispensable to the composition for ensuring good adhesiveness of the composition to teeth. The acid group-having polymerizable monomer is, for example, a polymerizable monomer having at least one acid group such as a phosphoric acid group, pyrophosphoric acid group, carboxylic acid group or sulfonic acid group and having a polymerizable unsaturated group such as an acryloyl group, methacryloyl group, vinyl group or styrene group. Examples of the compounds are mentioned below. In the invention, the expression "(meth)acryl" is to comprehensively include both methacryl and acryl.

The phosphoric acid group-having polymerizable monomer includes, for example, 2-(meth)acryloyloxyethyl dihydrogenphosphate, 3-(meth)acryloyloxypropyl dihydrogenphosphate, 4-(meth)acryloyloxybutyl dihydrogenphosphate, 5-(meth)acryloyloxypentyl dihydrogenphosphate, 6-(meth)acryloyloxyhexyl dihydrogenphosphate, 7-(meth)acryloyloxyheptyl dihydrogenphosphate, 8-(meth)acryloyloxyoctyl dihydrogenphosphate, 9-(meth)acryloyloxynonyl dihydrogenphosphate, 10-(meth)acryloyloxydecyl dihydrogenphosphate, 11-(meth)acryloyloxyundecyl dihydrogenphosphate, 12-(meth)acryloyloxydodecyl dihydrogenphosphate, 16-(meth)acryloyloxyhexadecyl dihydrogenphosphate, 20-(meth) acryloyloxyeicosyl dihydrogenphosphate, di[2-(meth)acryloyloxyethyl] hydrogenphosphate, di[4-(meth)acryloyloxybutyl] hydrogenphosphate, di[6-(meth)acryloyloxyhexyl] hydrogenphosphate, di[8-(meth)acryloyloxyoctyl] hydrogenphosphate, di[9-(meth)acryloyloxynonyl] hydrogenphosphate, di[10-(meth)acryloyloxydecyl] hydrogenphosphate, 1,3-di (meth)acryloyloxypropyl-2-dihydrogenphosphate, 2-(meth)acryloyloxyethylphenyl hydrogenphosphate, 2-(meth)acryloyloxyethyl-2'-bromoethyl hydrogenphosphate, 2-(meth)acryloyloxyethylphenyl phosphate; (5-methacryloxy)pentyl-3-phosphonopropionate, (6-methacryloxy)hexyl-3-phosphonopropionate, (10-methacryloxy)decyl-3-phosphonopropionate, (6-methacryloxy)hexyl-3-phosphonoacetate, (10-methacryloxy)decyl-3-phosphonoacetate, as in JP-A 294286/1991; 2-methacryloyloxyethyl(4-methoxyphenyl) hydrogenphosphate, 2-methacryloyloxypropyl(4-methoxyphenyl) hydrogenphosphate, as in JP-A 281885/1987; and other phosphoric acid group-having polymerizable monomers such as those exemplified in JP-A 113089/1977, 67740/1978, 69494/1978, 144939/1978, 128393/1983 and 192891/1993; and their acid chlorides.

The pyrophosphoric acid group-having polymerizable monomer includes, for example, di[2-(meth)acryloyloxyethyl] pyrophosphate, di[4-(meth)acryloyloxybutyl] pyrophosphate, di[6-(meth)acryloyloxyhexyl] pyrophosphate, di[8-(meth)acryloyloxyoctyl] pyrophosphate, di[10-(meth)acryloyloxydecyl] pyrophosphate, and their acid chlorides.

The carboxylic acid group-having polymerizable monomer includes, for example, maleic acid, methacrylic acid, 4-(meth)acryloyloxyethoxycarbonylphthalic acid, 4-(meth)acryloyloxybutyloxycarbonylphthalic acid, 4-(meth)acryloyloxyhexyloxycarbonylphthalic acid, 4-(meth)acryloyloxyoctyloxycarbonylphthalic acid, 4-(meth)acryloyloxydecyloxycarbonylphthalic acid, and their acid anhydrides; 5-(meth)acryloylaminopentylcarboxylic acid, 6-(meth)acryloyloxy-1,1-hexane-dicarboxylic acid, 8-(meth)acryloyloxy-1,1-octane-dicarboxylic acid, 10-(meth)acryloyloxy-1,1-decane-dicarboxylic acid, 11-(meth)acryloyloxy-1,1-undecane-dicarboxylic acid, and their acid chlorides.

The sulfonic acid group-having polymerizable monomer includes, for example, 2-(meth)acrylamido-2-methylpropanesulfonic acid, styrenesulfonic acid, 2-sulfoethyl (meth)acrylate. Of those, preferred are the phosphoric acid group-having polymerizable monomers, as the adhesiveness of the composition containing the monomer to teeth is extremely good.

One or more different types of such acid group-having polymerizable monomers may be used herein either singly or as combined. Regarding its blend ratio in the composition, if the amount of the acid group-having polymerizable monomer therein is too small or too large, the adhesiveness of the antibacterial composition to teeth will lower. Accordingly, the amount of the acid group-having polymerizable monomer in the composition may fall generally between 0.1 and 50 % by weight of the composition, but preferably between 1 and 40 % by weight, more preferably between 5 and 30 % by weight.

The hydrophilic polymerizable monomer (c) to be in the composition of the invention has a solubility in water at 25°C of at least 10 % by weight, preferably at least 30 % by weight. Concretely, it includes, for example, 2-hydroxyethyl (meth)acrylate, 2-hydroxypropyl (meth)acrylate, 3-hydroxypropyl (meth)acrylate, 1,3-dihydroxypropyl (meth)acrylate, 2,3-dihydroxypropyl (meth)acrylate, dipentaerythritol di(meth)acrylate, (meth)acrylamide, 2-hydroxyethyl(meth)acrylamide, and polyethylene glycol di(meth)acrylate (in which the number of the oxyethylene groups is at least 9).

One or more different types of such hydrophilic polymerizable monomers may be used herein either singly or as combined. Regarding its blend ratio in the composition, if the amount of the hydrophilic polymerizable monomer therein is too small or too large, the adhesiveness of the antibacterial composition to teeth will lower. Accordingly, the amount of the hydrophilic polymerizable monomer in the composition may fall generally between 5 and 95 % by weight of the composition, but preferably between 1.0 and 90 % by weight, more preferably between 15 and 70 % by weight.

Water (d) to be in the antibacterial composition of the invention must not substantially contain impurities that may have some negative influences on the antibacterial property of the composition and on the ability of the composition to bond a restorative material to teeth, for which, therefore, preferred is distilled water or ion-exchanged water. If the amount of water in the composition is too small or too large, the antibacteriality and the adhesiveness of the composition will lower. Therefore, the water content of the antibacterial composition may fall generally between 0.1 and 80 % by weight of the composition, but preferably between 1 and 75 % by weight, more preferably between 10 and 60 % by weight.

The antibacterial composition of the invention contains a basic compound (e) selected from alkali metal hydroxides, strong basic acid salts not having an aromatic group and aliphatic amines, for improving the antibacteriality and the storage stability of the composition. Preferably, the basic compound can form a water-soluble salt with the acid group-having polymerizable monomer in the composition. The solubility in water at 25°C of the salt is generally at least 5 % by weight. The alkali metal hydroxides include, for example, sodium hydroxide, lithium hydroxide and potassium hydroxide. For the strong basic acids not having an aromatic group, preferred are strong basic acid salts to be formed from alkali metals and weak acids having a pKa of at least 3, such as lithium carbonate, sodium carbonate, potassium carbonate, lithium hydrogencarbonate, sodium hydrogencarbonate, potassium hydrogencarbonate, sodium formate, sodium hydrogenoxalate, sodium acetate, potassium acetate, sodium propionate, sodium borate, sodium dihydrogenphosphite, potassium dihydrogenphosphite, sodium dihydrogenphosphate, potassium dihydrogenphosphate, disodium hydrogenphosphate, dipotassium hydrogenphosphate.

The aliphatic amines may be any of primary aliphatic amines, secondary aliphatic amines and tertiary aliphatic amines, but preferred are tertiary aliphatic amines. For the tertiary aliphatic amines, for example, preferred are trimethylamine, triethylamine, N-ethyldiethanolamine, N-n-butyldiethanolamine, N-lauryldiethanolamine, triethanolamine, (2-dimethylamino)ethyl methacrylate, (2-diethylamino)ethyl methacrylate, (2-dipropylamino)ethyl methacrylate, (3-dimethylamino)propyl methacrylate, (4-dimethylamino)butyl methacrylate, (6-dimethylamino)hexyl methacrylate, (6-diethylamino)hexyl methacrylate, (10-dimethylamino)decyl methacrylate, N-methyldiethanolamine dimethacrylate, triethanolamine dimethacrylate, and triethanolamine trimethacrylate.

The aliphatic amines are especially preferred, as they form stable water-soluble salts with the acid group-having polymerizable monomer, especially with the phosphoric acid group-having polymerizable monomer in the composition, and the salts are also highly adhesive to teeth. More preferred are polymerizable group-having aliphatic tertiary amines such as (2-dimethylamino)ethyl methacrylate, (2-diethylamino)ethyl methacrylate, (2-dipropylamino)ethyl methacrylate, (6-diethylamino)hexyl methacrylate, (6-dimethylamino)hexyl methacrylate, N-methyldiethanolamine dimethacrylate, and triethanolamine dimethacrylate.

One or more different types of such basic compounds may be used herein either singly or as combined. The

blend ratio of the compound in the antibacterial composition is not specifically defined. However, if the amount of the compound therein is too small, the antibacteriality and the storage stability of the composition will be poor; but if too large, the adhesiveness thereof will lower. Accordingly, the amount of the basic compound in the composition may fall generally between 0.01 and 20 % by weight of the composition, but preferably between 0.5 and 10 % by weight, more preferably between 2 and 7 % by weight. Also preferably, the blend ratio of the basic compound in the composition is so controlled that the pH of the composition may fall between 1.5 and 4.5, more preferably between 1.8 and 3.5.

Regarding its use, when the antibacterial composition of the invention is, after applied onto a tooth, thinned with a dental air syringe, it can be cured along with the material to overlay it such as a dental bonding material, a dental cement material, a dental composite or the like. Therefore, the composition does not always require a polymerization initiator. However, when a dental operator has applied too much the antibacterial composition of the invention onto a tooth to be treated with it and when the excess composition has therefore remained on the tooth, or when the composition is used as an adhesive layer to have a thickness of 5 μm or more, a polymerization initiator is preferably added to the composition for ensuring or improving the bonding strength of the coated composition.

The polymerization initiator may be any known one, including, for example, α-diketones, ketals, thioxanthones, acylphosphine oxides, coumarins, halomethyl-substituted-s-triazine derivatives, and organic peroxides.

The α-diketones include, for example, camphorquinone, benzil and 2,3-pentanedione. The ketals include, for example, benzyldimethyl ketal, benzyldiethyl ketal. The thioxanthones include, for example, 2-chlorothioxanthone, 2,4-diethylthioxanthone. The acylphosphine oxides include, for example, 2,4,6-trimethylbenzoyldiphenylphosphine oxide, 2,6-dimethoxybenzoyldiphenylphosphine oxide, 2,6-dichlorobenzoyldiphenylphosohine oxide, 2,3,5,6-tetramethylbenzoyldiphenylphosphine oxide, benzoyl-di-(2,6-dimethylphenyl) phosphonate, 2,4,6-trimethylbenzoylethoxyphenylphosphine oxide, and water-soluble acylphosphine oxide compounds such as those described in JP-B 57916/1991.

The coumarins are, for example, those described in JP-A 245525/1998, such as 3,3'-carbonylbis(7-diethylamino)coumarin, 3-(4-methoxybenzoyl)coumarin, 3-thienoylcoumarin. The halomethyl-substituted-s-triazine derivatives are, for example, those described in JP-A 245525/1998, such as 2,4,6-tris(trichloromethyl)-s-triazine, 2,4,6-tris(tribromomethyl)-s-triazine, 2-methyl-4,6-bis(trichloromethyl)-s-triazine.

The organic peroxides include, for example, diacyl peroxides, peroxyesters, dialkyl peroxides, peroxyketals, ketone peroxides, hydroperoxides. Concretely, the diacyl peroxides include, for example, benzoyl peroxide, 2,4-dichlorobenzoyl peroxide, m-toluoyl peroxide. The peroxyesters include, for example, t-butyl peroxybenzoate, bis-t-butyl peroxyisophthalate, 2,5-dimethyl-2,5-bis(benzoylperoxy)hexane, t-butylperoxy-2-ethyl hexanoate, t-butylperoxyisopropyl carbonate. The dialkyl peroxides include, for example, dicumyl peroxide, di-t-butyl peroxide, lauroyl peroxide. The peroxyketals include, for example, 1,1-bis(t-butylperoxy)-3,3,5-trimethylcyclohexane, 1,1-bis(t-butylperoxy)cyclohexane, 1,1-bis(t-hexylperoxy)cyclohexane. The ketone peroxides include, for example, methyl ethyl ketone peroxide, cyclohexanone peroxide, methyl acetate peroxide. The hydroperoxides include, for example, t-butyl hydroperoxide, cumene hydroperoxide, p-diisopropylbenzene peroxide.

One or more different types of such polymerization initiators may be used herein either singly or as combined. The amount of the polymerization initiator to be in the antibacterial composition of the invention may fall generally between 0.01 and 10 % by weight of the composition, but preferably between 0.05 and 5 % by weight, more preferably between 0.1 and 3 % by weight.

If desired, the antibacterial composition of the invention may further contain any other polymerizable monomer, in addition to the above-mentioned, acid group-having polymerizable monomer and hydrophilic polymerizable monomer, for the purpose of improving the adhesiveness, the curability and the mechanical strength of the composition. The additional polymerizable monomer includes, for example, esters of α-cyanoacrylic acid, (meth)acrylic acid, α-halogenoacrylic acids, crotonic acid, cinnamic acid, sorbic acid, maleic acid and itaconic acid; and (meth)acrylamide derivatives, vinyl esters, vinyl ethers, mono-N-vinyl derivatives, and styrene derivatives. Of those, preferred for use herein are (meth) acrylates.

Examples of the polymerizable monomers usable in the invention are mentioned below, of which those having one olefinic double bond are referred to as monofunctional monomers, and those having two or more olefinic bonds are referred to as difunctional monomers, trifunctional monomers, etc., depending on the number of the olefinic double bonds therein.

Monofunctional Monomers:

Methyl (meth)acrylate, ethyl (meth)acrylate, propyl (meth)acrylate, isopropyl (meth)acrylate, butyl (meth)acrylate, isobutyl (meth)acrylate, benzyl (meth)acrylate, lauryl (meth)acrylate, 2,3-dibromopropyl (meth)acrylate, 3-methacryloyloxypropyltrimethoxysilane, 11-methacryloyloxyundecyltrimethoxysilane.

Difunctional Monomers:

Ethylene glycol di(meth)acrylate, triethylene glycol di(meth)acrylate, propylene glycol di(meth)acrylate, neopentylglycol di(meth)acrylate, 1,6-hexanediol di(meth)acrylate, 1,10-decanediol di(meth)acrylate, bisphenol A diglycidyl (meth)acrylate, 2,2-bis[4-(meth)acryloyloxyethoxyphenyl]propane, 2,2-bis[4-(meth)acryloyloxypolyethoxyphenyl]propane, 2,2-bis[4-[3-(meth)acryloyloxy-2-hydroxypropoxy]phenyl]propane, 1,2--bis[3-(meth)acryloyloxy-2-hydroxypropoxy]ethane, pentaerythritol di(meth)acrylate, 1,2-bis(3-methacryloyloxy-2-hydroxypropoxy)ethane, [2,2,4-trimethylhexamethylenebis(2-carbamoyloxyethyl)] dimethacrylate, 1,3-di(meth)acryloyloxy-2-hydroxypropane.

Trifunctional and Higher Polyfunctional Monomers:

Trimethylolpropane tri(meth)acrylate, trimethylolethane tri(meth)acrylate, tetramethylolmethane tri(meth)acrylate, pentaerythritol tetra(meth)acrylate, N,N'-(2,2,4-trimethylhexamethylene)bis[2-(aminocarboxy)propane-1,3-diol] tetramethacrylate, 1,7-diacryloyloxy-2,2,6,6-tetraacryloyloxymethyl-4-oxyheptane.

One or more different types of these additional polymerizable monomers may be used herein either singly or as combined. However, if the amount of the additional polymerizable monomer is too much therein, the adhesive strength of the antibacterial composition to teeth will lower. Therefore, the amount may be generally at most 50 % by weight of the composition, but preferably at most 30 % by weight.

Further if desired, the antibacterial composition of the invention may also contain a volatile organic solvent for the purpose of assisting the dissolution of the antibacterial salt compound, the acid group-having polymerizable monomer, the hydrophilic polymerizable monomer and the polymerization initiator that constitute the composition. For the volatile organic solvent, generally preferred are those having a boiling point of not higher than 150°C, more preferably not higher than 100°C under atmospheric pressure. Preferred examples of the solvent of the type are alcohols such as ethanol, methanol, 1-propanol, isopropyl alcohol; ketones such as acetone, methyl ethyl ketone; ester compounds such as ethyl acetate, methyl acetate, ethyl propionate; ethers such s 1,2-dimethoxyethane, 1,2-diethoxyethane, tetrahydrofuran; hydrocarbon compounds such as heptane, hexane, toluene; and halogenohydrocarbon compounds such as chloroform, dichloromethane. Of those, especially preferred are water-soluble volatile solvents such as ethanol and acetone.

One or more different types of such volatile organic solvents may be used herein either singly or as combined. The amount of the volatile organic solvent to be in the antibacterial composition may be generally at most 50 % by weight of the composition, but preferably at most 30 % by weight. Desirably, the volatile solvent, if any, in the antibacterial composition of the invention is, after the composition has been applied to teeth, evaporated away by the use of a dental syringe in order that it does not interfere with the adhesiveness of the composition.

Still optionally, the antibacterial composition of the invention may further contain a polymerization inhibitor, a colorant, a fluorescent agent, and an UV absorbent. It may also contain any known fluorine compound capable of releasing fluoride ions, such as sodium fluoride, lithium fluoride, sodium monofluorophosphate, and cetylamine hydrofluoride, for the purpose of ensuring the acid resistance of the teeth that have received the composition.

In addition, the antibacterial composition of the invention may also contain a filler for improving the handlability, the coatability, the flowability and the mechanical strength of the composition. The filler may be any of organic, inorganic or even composite fillers. The inorganic fillers include, for example, silica, silica-based minerals such as kaolin, clay, mica; and silica-based ceramics and glass additionally containing any of $Al_2O_3$, $B_2O_3$, $TiO_2$, $ZrO_2$, BaO, $La_2O_3$, $SrO_2$, CaO, $P_2O_5$. Especially preferred are lanthanum glass, barium glass, strontium glass, soda glass, lithium borosilicate glass, zinc glass, fluoroaluminium borosilicate glass, borosilicate glass, bioglass. Also preferred are crystalline quartz, hydroxyapatite, alumina, titanium oxide, yttrium oxide, zirconia, calcium phosphate, barium sulfate, aluminium hydroxide. The organic fillers may be of organic resin, including, for example, polymethyl methacrylate, polymers of polyfunctional methacrylates, polyamides, polystyrenes, polyvinyl chloride, chloroprene rubber, nitrile rubber, styrene-butadiene rubber. Also employable herein are inorganic/organic composite fillers, which may be prepared by dispersing an inorganic filler in an organic resin, or by coating an inorganic filler with an organic resin such as that mentioned above.

If desired, the fillers may be previously subjected to surface treatment with any known surface-treating agent such as silane coupling agent. The surface-treated fillers are effective for controlling and enhancing the handlability, the coatability, the flowability and the mechanical strength of the composition. The surface-treating agent includes, for example, vinyltrimethoxysilane, vinyltriethoxysilane, vinyltrichlorosilane, vinyltri(β-methoxyethoxy)silane, γ-methacryloyloxypropyltrimethoxysilane, γ-glycidoxypropyltrimethoxysilane, γ-mercaptopropyltrimethoxysilane, γ-aminopropyltriethoxysilane.

One or more different types of these fillers may be used herein either singly or as combined. The amount of the filler, if any, in the antibacterial composition of the invention may be generally at most 30 % by weight of the composition, but preferably at most 15 % by weight. More preferably, the filler has a mean particle size of from 0.001 to 50 μm, and it is uniformly dispersed in the composition

The antibacterial composition of the invention generally serves as an adhesive primer, which is for preventing

the cariogenic bacteria in teeth from propagating, or for killing them, and for enhancing the adhesiveness of dental bonding materials such as resin cement, glass ionomer cement, zinc phosphate cement, polycarboxylate cement, silicate cement and other bonding ingredients. In addition, the composition is also usable as a fissure sealant for pit fissures, a coating agent for root surfaces and neighboring teeth portions, and an adhesive primer or adhesive agent for bonding a dental composite resin or a filler compomer to teeth.

The antibacterial composition of the invention is applicable to not only hard tissues such as teeth but also to crown restorative materials such as metals, ceramics, cured composites. In addition, it may be combined with any of commercially-available metal primers for dental use, ceramic-bonding primers, acid etchants, and tooth cleaners such as hypochlorites.

The invention is described in more detail with reference to the following Examples, which, however, are not intended to restrict the scope of the invention. The meanings of the abbreviations and nomenclatures used herein are mentioned below.

[Antibacterial Salt Compounds]

CPC: cetylpyridinium chloride
MDPB: 12-methacryloyloxydodecylpyridinium bromide
MHPC: 16-methacryloyloxyhexadecylpyridinium chloride
MHAC: 16-methacryloyloxyhexadecyltrimethylammonium chloride
MPHC: 16-methacryloyloxyhexadecyltrimethylphosphonium chloride
TMBEA: trimethylbenzylammonium chloride

[Acid Group-having Polymerizable Monomers]

MDP: 10-methacryloyloxydecyl dihydrogenphosphate
MEPP: 2-methacryloyloxyethylphenylphosphonic acid
MA: maleic acid

[Hydrophilic Polymerizable Monomers]

HEMA: 2-hydroxyethyl methacrylate
9G: polyethylene glycol dimethacrylate (in which the number of oxyethylene groups is 9)

[Basic Compounds]

DMAEMA: (2-dimethylamino)ethyl methacrylate
DEAHMA: (6-diethylamino)hexyl methacrylate
DPAEMA: (2-dipropylamino)ethyl methacrylate
TEA: triethanolamine
$NaHCO_3$: sodium hydrogencarbonate
LiOH: lithium hydroxide
$H_2KPO_4$: potassium dihydrogenphosphate
DEPT: N,N-di(2-hydroxyethyl)-p-toluidine
DMAB: 4-N,N-dimethylaminobenzophenone
DMPT: N,N-dimethyl-p-toluidine

[Polymerization Initiators]

CQ: camphorquinone
TMDPO: 2,4,6-trimethylbenzoyldiphenylphosphine oxide

[Others]

BHT: 2,6-di-t-butylhydroxytoluene
GDM: 1,3-dimethacryloyloxy-2-hydroxypropane
PDM: 1,2-bis(3-methacryloyloxy-2-hydroxypropoxy)ethane
RE-106: Red #106 (pigment)
BU-1: Blue #1 (pigment)

Example 1:

An antibacterial composition comprising MDPB (5 parts by weight), MDP (15 parts by weight), HEMA (40 parts by weight), distilled water (40 parts by weight) and DMAEMA (3 parts by weight) was prepared. This was tested for the antibacterial property, the adhesiveness and the storage stability, according to the antibacterial test, the bonding test and the storage stability test mentioned below. The results are given in Table 1.

[Antibacterial Test]

1 g of bovine dentin powder that had been previously sterilized and dried, 0.5 ml of the antibacterial composition of Example 1, and 0.5 ml of aqueous 50 % HEMA solution were put into a sample tube, stirred for 10 minutes, and then centrifuged to collect the upper liquid phase. This is a 50 % sample liquid. The 50 % sample liquid was diluted with sterilized water to prepare different samples having a concentration of 20 %, 10 %, 5 %, 2 % and 1 %. On the other hand, cells of *Streptococcus mutans* (IFO13955) that had been pre-incubated for 18 hours in a liquid brain heart infusion (BHI) medium (from Nippon Pharmaceutical) were diluted with germ-free water to prepare a cell dilution having a cell concentration of $2 \times 10^6$ (CFU/ml). The samples having different concentrations as above were tested with the cell dilution for the antibacterial property.

Concretely, 100 μl of each sample and 100 μl of the cell dilution were rapidly mixed on a micro-plate. After 20 seconds, the resulting mixture was diluted with BHI to 1/1000. 100 μl of the 1/1000 dilution was metered, applied onto a BHI-agar medium (from Nippon Pharmaceutical) plate that had been separately prepared, and uniformly spread thereover with a Conradi rod. With that, the plate was put in a thermostat at 37°C and the cells on the plate were aerobically incubated therein for 48 hours. For control, germ-free water alone not containing the antibacterial composition was tested in the same manner as above. The number of colonies formed in the BHI-agar medium was counted, and the cell death percentage was calculated according to the following equation:

$$\text{Cell Death Percentage (\%)} = \{[\text{number of colonies (in control)} - \text{number of colonies (in antibacterial composition-containing sample)}]/\text{number of colonies (in control)}\} \times 100$$

[Bonding Test]

A bovine anterior tooth was polished in wet with #1000 Silicon Carbide Abrasive Paper (from Nippon Abrasive Paper) to make its surface smooth, then its enamel or dentin was exposed out, and water existing on its surface was blown off with a dental air syringe. An adhesive tape (thickness: about 150 microns) with a hole having a diameter of 3 mm was stuck on the surface of the exposed enamel or dentin. The antibacterial composition of Example 1 was applied to the holed area with a brush, then left as such for 30 seconds, and dried with an air syringe until the antibacterial

composition was no more fluid. Next, a photopolymerizable, dental bonding material "Clearfilmegabond" (from Kuraray) was applied over it also with a brush to form thereon a layer having a thickness of about 100 μm, With that, this was exposed to light for 10 seconds and cured, for which used was a dental light emitter "Litel II" (from Gunma Ushio Electric). Next, a commercially-available, photopolymerizable dental composite resin, "Clearfill AP-X" (from Kuraray) was put on it, covered with a film of Eval® (from Kuraray), and pressed against a glass slide superposed thereon. In that condition, this was exposed to light for 40 seconds and cured, for which was used the same light emitter as above.

A stainless steel rod was attached to the cured surface with a commercially-available dental resin cement, "Panavia 21" (from Kuraray) being disposed therebetween. After left as such for 30 minutes, the test piece was dipped in water at 37°C for 24 hours, and then its bonding strength was measured. For the measurement, used was a universal tester (from Instron). At a cross head speed of 2 mm/min, the tensile bonding strength of the test piece was measured. Eight test pieces were prepared and tested under the same condition for their bonding strength, and their data were averaged.

[Storage Stability Test]

(1) Discoloration Test:

The antibacterial composition of Example 1 was stored in a thermostat at 50°C for 1 month. 200 μl of it was put into a colorless transparent glass chamber having a diameter of 1.4 mm, a depth of 2 mm and a thickness of 1 mm, and its values L* and b* were measured with a colorimeter (from Nippon Denshoku Kogyo). Not stored, a fresh sample of the composition was also measured in the same manner. From the data, obtained were ΔL* and Δb*. In addition, the stored sample was visually checked for discoloration.

(2) Bonding Test:

The antibacterial composition of Example 1 was stored in a thermostat at 50°C for 1 month. In the same bonding test as above, the thus-stored sample was tested for the tensile bonding strength to bovine dentin.

Examples 2 and 3:

Antibacterial compositions were prepared in the same manner as in Example 1, in which, however, MDPB was not used but CPC (Reference Example) or MHPC was used in place of it. These were tested for the antibacterial property, the adhesiveness and the storage stability in the same manner as in Example 1. The test data are given in Table 1.

Comparative Examples 1 to 3:

Antibacterial compositions were prepared in the same manner as in Examples 1 to 3, in which, however, DMAEMA was not used but a basic compound, aromatic amine (DEPT) was used in place of it. These were tested for the antibacterial property, the adhesiveness and the storage stability in the same manner as in Example 1. The test data are given in Table 1.

Comparative Example 4:

An antibacterial composition was prepared in the same manner as in Example 1, in which, however, DMAEMA was not used. This was tested for the antibacterial property, the adhesiveness and the storage stability in the same manner as in Example 1. The test data are given in Table 1.

Comparative Example 5:

An antibacterial composition was prepared in the same manner as in Example 1, in which, however, MDPB was not used. This was tested for the antibacterial property, the adhesiveness and the storage stability in the same manner as in Example 1. The test data are given in Table 1.

Table 1

| | | Blend Ratio (wt.%) | | | | | | | |
|---|---|---|---|---|---|---|---|---|---|
| | | Example 1 | Example 2 (Reference) | Example 3 | Comp.Ex.1 | Comp.Ex. 2 | Comp.Ex. 3 | Comp.Ex. 4 | Comp.Ex. 5 |
| Antibacterial Composition | MDPB | 5 | - | - | 5 | - | - | 5 | - |
| | CPC | - | 5 | - | - | 5 | - | - | - |
| | MHPC | - | - | 5 | - | - | 5 | - | - |
| | MDP | 15 | 15 | 15 | 15 | 15 | 15 | 15 | 15 |
| | HEMA | 40 | 40 | 40 | 40 | 40 | 40 | 40 | 40 |
| | Distilled water | 40 | 40 | 40 | 40 | 40 | 40 | 40 | 40 |
| | DMAEMA | 3 | 3 | 3 | - | - | - | - | 3 |
| | DEPT | - | - | - | 3 | 3 | 3 | - | - |
| ①Antibacterial Property (Cell Death Percentage : %) | | | | | | | | | |
| Concentration | 20% | 100 | 100 | 100 | 100 | 100 | 100 | 100 | 34 |
| | 10% | 100 | 100 | 100 | 100 | 100 | 100 | 100 | 30 |
| | 5% | 100 | 100 | 100 | 87 | 98 | 100 | 67 | 7 |
| | 2% | 100 | 100 | 100 | 27 | 55 | 67 | 17 | 5 |
| | 1% | 85 | 95 | 100 | 6 | 25 | 37 | 4 | 0 |
| ②Adhesiveness (MPa) | | | | | | | | | |
| | Bovine enamel | 19.1 | 15.6 | 20.1 | 19.0 | 15.6 | 19.1 | 18.9 | 19.6 |
| | Bovine dentin | 18.2 | 15.8 | 19.8 | 18.1 | 14.8 | 18.8 | 14.2 | 17.0 |
| ③Storage Stability | | | | | | | | | |
| Discoloration | ΔL* | 0.6 | 0.7 | 0.9 | 8.8 | 9.1 | 9.3 | 0.7 | 0.6 |
| | Δb* | 1.6 | 1.7 | 1.9 | 12.2 | 13.3 | 14.5 | 1.8 | 1.4 |
| | Visual check | colorless | colorless | colorless | dark brown | dark brown | dark brown | colorless | colorless |

(continued)

| ③Storage Stability | | | | | | | | | |
|---|---|---|---|---|---|---|---|---|---|
| Adhesiveness | Bovine dentin | 17.8 | 14.1 | 18.1 | 10.8 | 9.7 | 11.6 | 7.0 | 14.7 |

As is obvious from Table 1, the antibacterial compositions prepared by mixing an antibacterial pyridinium salt compound, MDP, HEMA, distilled water and DMAEMA (Examples 1 to 3) completely killed the cells of *Streptococcus mutans* even when their concentration was 2 %. In addition, the adhesiveness of these antibacterial compositions was good; and even after stored in a thermostat at 50°C for 1 month, the compositions did not discolor when observed visually, and their bonding strength to dentin lowered little. As opposed to these, however, the antibacterial compositions containing a basic compound, aromatic amine DEPT (Comparative Examples 1 to 3) could not completely kill the cells of *Streptococcus mutans* when their concentration was 2 %. In addition, when stored in a thermostat at 50°C for 1 month, they greatly discolored from colorless to dark brown and their bonding strength to dentin greatly lowered. The antibacterial composition not containing a basic compound (Comparative Example 4) could not also completely kill the cells of *Streptococcus mutans* even when its concentration was 5 %. In addition, when stored in a thermostat at 50°C for 1 month, the bonding strength of the composition to dentin greatly lowered. The antibacterial composition not containing an antibacterial pyridinium salt compound (Comparative Example 5) could not completely kill the cells of *Streptococcus mutans* even when its concentration was 20 %.

Examples 4 to 9, Comparative Examples 6 and 7:

As in Table 2, different antibacterial compositions comprising MDPB, MDP, HEMA, distilled water, TMDPO and a basic compound were prepared. These were tested for the antibacterial property, the adhesiveness and the storage stability in the same manner as in Example 1. The test data are given in Table 2.

Comparative Example 8:

An antibacterial composition was prepared in the same manner as in Example 4, which, however, did not contain DMAEMA. This was tested for the antibacterial property, the adhesiveness and the storage stability in the same manner as in Example 1. The test data are given in Table 2.

Table 2

| | | Blend Ration (wt.%) | | | | | | | | |
|---|---|---|---|---|---|---|---|---|---|---|
| | | Example 4 | Example 5 | Example 6 | Example 7 | Example 8 | Example 9 | Comp.Ex. 6 | Comp.Ex. 7 | Comp.Ex. 8 |
| Antibacterial Composition | MDPB | 3 | 3 | 3 | 3 | 3 | 3 | 3 | 3 | 3 |
| | MDP | 10 | 10 | 10 | 10 | 10 | 10 | 10 | 10 | 10 |
| | HEMA | 43.5 | 43.5 | 43.5 | 43.5 | 43.5 | 43.5 | 43.5 | 43.5 | 43.5 |
| | Distilled water | 43.5 | 43.5 | 43.5 | 43.5 | 43.5 | 43.5 | 43.5 | 43.5 | 43.5 |
| | TMDPO | 0.1 | 0.1 | 0.1 | 0.1 | 0.1 | 0.1 | 0.1 | 0.1 | 0.1 |
| | DMAEMA | 2 | - | - | - | - | - | - | - | - |
| | DEAHMA | - | 2 | - | - | - | - | - | - | - |
| | TEA | - | - | 2 | - | - | - | - | - | - |
| | $NaHCO_3$ | - | - | - | 2 | - | - | - | - | - |
| | LiOH | - | - | - | - | 2 | - | - | - | - |
| | $H_2KPO_4$ | - | - | - | - | - | 2 | - | - | - |
| | DEPT | - | - | - | - | - | - | 2 | - | - |
| | DMAB | - | - | - | - | - | - | - | 2 | - |
| ①Antibacterial Property (Cell Death Percentage : %) | | | | | | | | | | |
| Concentration | 20% | 100 | 100 | 100 | 100 | 100 | 100 | 100 | 100 | 100 |
| | 10% | 100 | 100 | 100 | 100 | 100 | 100 | 100 | 100 | 91 |
| | 5% | 100 | 100 | 100 | 100 | 100 | 100 | 71 | 80 | 68 |
| | 2% | 98 | 96 | 82 | 91 | 90 | 88 | 28 | 58 | 48 |
| | 1% | 71 | 75 | 66 | 70 | 72 | 68 | 8 | 10 | 12 |
| ②Adhesiveness (MPa) | | | | | | | | | | |
| | Bovine enamel | 17.8 | 17.1 | 15.8 | 15.9 | 15.6 | 15.1 | 17.5 | 17.4 | 18.1 |
| | Bovine dentin | 17.6 | 18.3 | 14.6 | 15.0 | 15.1 | 15.2 | 16.6 | 16.3 | 15.6 |

(continued)

| ③Storage Stability (at 50°C for 1 month) Discoloration ΔL* 0.3 0.4 | | | | | | | | | | |
|---|---|---|---|---|---|---|---|---|---|---|
| Discoloration | ΔL* | 0.3 | 0.4 | 0.4 | 0.3 | 0.5 | 0.5 | 6.1 | 6.3 | 0.4 |
| | Δb* | 0.8 | 0.9 | 0.8 | 0.9 | 1.0 | 0.8 | 8.3 | 14.5 | 1.0 |
| | Visual check | colorless | colorless | colorless | colorless | colorless | colorless | dark brown | dark brown | colorless |
| Adhesiveness | Bovine dentin | 17.5 | 17.8 | 14.1 | 14.4 | 14.3 | 14.2 | 8.6 | 9.6 | 6.9 |

As is obvious from Table 2, the antibacterial compositions comprising MDPB, MDP, HEMA, distilled water, TMDPO, and any of an aliphatic amine, an alkali metal hydroxide or a strong basic acid salt (Examples 4 to 9) completely killed the cells of *Streptococcus mutans* even when the amount of MDPB therein was about 3 % by weight of the composition and the concentration of the composition was 5 %. In addition, the adhesiveness of these antibacterial compositions was good; and even after stored in a thermostat at 50°C for 1 month, the compositions did not discolor when observed visually, and their bonding strength to dentin lowered little. As opposed to these, however, the antibacterial compositions containing a basic compound, aromatic amine DEPT or DMAB (Comparative Examples 6 and 7) could not completely kill the cells of *Streptococcus mutans* when their concentration was 5 %. In addition, when stored in a thermostat at 50°C for 1 month, they greatly discolored from colorless to dark brown and their bonding strength to dentin greatly lowered. The antibacterial composition not containing a basic compound (Comparative Example 8) could not also completely kill the cells of *Streptococcus mutans* even when its concentration was 10 %. In addition, when stored in a thermostat at 50°C for 1 month, the bonding strength of the composition to dentin greatly lowered.

Examples 10 to 13:

As in Table 3, different antibacterial compositions comprising MHPC, an acid group-having polymerizable monomer, a hydrophilic polymerizable monomer, distilled water, CQ, BHT and DPAEMA were prepared. These were tested for the antibacterial property, the adhesiveness and the storage stability in the same manner as in Example 1. The test data are given in Table 3.

Comparative Examples 9 to 12:

Different antibacterial compositions were prepared in the same manner as in Examples 10 to 13, which, however, did not contain DPAEMA but contained an aromatic amine DEPT in place of it. These were tested for the antibacterial property, the adhesiveness and the storage stability in the same manner as in Example 1. The test data are given in Table 3.

Comparative Example 13:

An antibacterial composition was prepared in the same manner as in Example 10, which, however, did not contain a basic compound (DPAEMA). This was tested for the antibacterial property, the adhesiveness and the storage stability in the same manner as in Example 1. The test data are given in Table 3.

Table 3

| | | Blend Ration (wt.%) | | | | | | | | |
|---|---|---|---|---|---|---|---|---|---|---|
| | | Example 10 | Example 11 | Example 12 | Example 13 | Comp.Ex. 9 | Comp.Ex. 10 | Comp.Ex. 11 | Comp.Ex. 12 | Comp.Ex. 13 |
| Antibacterial Composition | MHPC | 7 | 7 | 7 | 7 | 7 | 7 | 7 | 7 | 7 |
| | MDP | 20 | - | - | 20 | 20 | - | - | 20 | 20 |
| | MEPP | - | 20 | - | - | - | 20 | - | - | - |
| | MA | - | - | 20 | - | - | - | 20 | - | - |
| | HEMA | 36.5 | 36.5 | 36.5 | - | 36.5 | 36.5 | 36.5 | - | 36.5 |
| | 9G | - | - | - | 36.5 | - | - | - | 36.5 | - |
| | Distilled water | 36.5 | 36.5 | 36.5 | 36.5 | 36.5 | 36.5 | 36.5 | 36.5 | 36.5 |
| | CQ | 0.2 | 0.2 | 0.2 | 0.2 | 0.2 | 0.2 | 0.2 | 0.2 | 0.2 |
| | BHT | 0.05 | 0.05 | 0.05 | 0.05 | 0.05 | 0.05 | 0.05 | 0.05 | 0.05 |
| | DPAEMA | 4 | 4 | 4 | 4 | - | - | - | - | - |
| | DEPT | - | - | - | - | 4 | 4 | 4 | 4 | - |
| ①Antibacterial Property (Cell Death Percentage : %) | | | | | | | | | | |
| Concentration | 20% | 100 | 100 | 100 | 100 | 100 | 100 | 100 | 100 | 100 |
| | 10% | 100 | 100 | 100 | 100 | 100 | 100 | 100 | 100 | 100 |
| | 5% | 100 | 100 | 100 | 100 | 100 | 100 | 100 | 100 | 80 |
| | 2% | 100 | 100 | 100 | 100 | 70 | 78 | 88 | 78 | 68 |
| | 1% | 100 | 100 | 100 | 100 | 50 | 40 | 46 | 50 | 35 |
| ②Adhesiveness (MPa) | | | | | | | | | | |
| | Bovine enamel | 19.5 | 20.8 | 15.8 | 19.1 | 19.4 | 18.8 | 14.9 | 18.5 | 18.8 |
| | Bovine dentin | 18.2 | 18.0 | 15.6 | 18.1 | 17.7 | 17.0 | 14.6 | 17.1 | 14.6 |

(continued)

| | | | | | | | | | | |
|---|---|---|---|---|---|---|---|---|---|---|
| ③Storage Stability | | | | | | | | | | |
| Discoloration | ΔL* | 0.8 | 0.9 | 0.5 | 0.9 | 13.1 | 12.3 | 11.1 | 12.3 | 0.8 |
| | Δb* | 2.1 | 2.3 | 1.6 | 2.0 | 17.3 | 18.5 | 16.3 | 17.5 | 2.6 |
| | Visual check | pale yellow | pale yellow | pale yellow | pale yellow | dark brown | dark brown | dark brown | dark brown | pale yellow |
| Adhesiveness | Bovine dentin | 17.8 | 17.6 | 14.7 | 17.7 | 9.6 | 10.6 | 8.6 | 11.6 | 6.7 |

As is obvious from Table 3, the antibacterial compositions comprising MHPC, an acid group-having polymerizable monomer, a hydrophilic polymerizable monomer, distilled water, CQ, BHT and DPAEMA (Examples 10 to 13) completely killed the cells of *Streptococcus mutans* even when their concentration was 1 %. In addition, the adhesiveness of these antibacterial compositions was good; and even after stored in a thermostat at 50°C for 1 month, the compositions still kept pale yellow owing to CQ existing therein, and did not discolor when observed visually, and their bonding strength to dentin lowered little. As opposed to these, however, the antibacterial compositions containing a basic compound, aromatic amine DEPT (Comparative Examples 9 to 12) could not completely kill the cells of *Streptococcus mutans* when their concentration was 2 %. In addition, when stored in a thermostat at 50°C for 1 month, they greatly discolored from pale yellow to dark brown and their bonding strength to dentin greatly lowered. The antibacterial composition not containing a basic compound (Comparative Example 13) could not also completely kill the cells of *Streptococcus mutans* even when its concentration was 5 %. In addition, when stored in a thermostat at 50°C for 1 month, the bonding strength of the composition to dentin greatly lowered.

Examples 14 to 18:

As in Table 4, different antibacterial compositions comprising an antibacterial salt compound of MHAC, MPHC, MDPB or TMBEA (Reference Example), an acid group-having polymerizable monomer of MDP, a hydrophilic polymerizable monomer of HEMA, a hydrophobic polymerizable monomer of GDM or PDM, distilled water, TMDPO, BHT, a pigment (RE-106 or BU-1), and a basic compound of DMAEMA were prepared. These were tested for the antibacterial property, the adhesiveness and the storage stability in the same manner as in Example 1. The test data are given in Table 4.

Comparative Examples 14 to 17:

Different antibacterial compositions were prepared in the same manner as in Example 14, 15, 17 or 18, which, however, did not contain DMAEMA but contained an aromatic amine DEPT or DMPT in place of it. These were tested for the antibacterial property, the adhesiveness and the storage stability in the same manner as in Example 1. The test data are given in Table 4.

Table 4

| | | Blend Ration (wt.%) | | | | | | | | |
|---|---|---|---|---|---|---|---|---|---|---|
| | | Example 14 | Example 15 | Example 16 | Example 17 (Reference) | Example 18 | Comp.Ex. 14 | Comp.Ex. 15 | Comp.Ex. 16 | Comp.Ex. 17 |
| Antibacterial Composition | MHAC | 5 | - | - | - | - | 5 | - | - | - |
| | MPHC | - | 5 | - | - | - | - | 5 | - | - |
| | MDPB | - | - | 5 | - | - | - | - | 5 | - |
| | TMBEA | - | - | - | 5 | - | - | - | - | 5 |
| | MDP | 20 | 20 | 20 | 20 | 20 | 20 | 20 | 20 | 20 |
| | HEMA | 35 | 35 | 35 | 35 | 35 | 35 | 35 | 35 | 35 |
| | GDM | 10 | 10 | 10 | 10 | - | 10 | 10 | 10 | - |
| | PDM | - | - | - | - | 10 | - | - | - | 10 |
| | Distilled water | 30 | 30 | 30 | 30 | 30 | 30 | 30 | 30 | 30 |
| | TMDPO | 0.2 | 0.2 | 0.2 | 0.2 | 0.2 | 0.2 | 0.2 | 0.2 | 0.2 |
| | BHT | 0.05 | 0.05 | 0.05 | 0.05 | 0.05 | 0.05 | 0.05 | 0.05 | 0.05 |
| | RE-106 | trace | trace | trace | - | - | trace | trace | - | - |
| | BU-1 | - | - | - | trace | trace | - | - | trace | trace |
| | DMAEMA | 4 | 4 | 4 | 4 | 4 | - | - | - | - |
| | DEPT | - | - | - | - | - | 4 | 4 | 4 | - |
| | DMPT | - | - | - | - | - | - | - | - | 4 |

(continued)

| | | | | | | | | | | |
|---|---|---|---|---|---|---|---|---|---|---|
| ①Antibacterial Property (Cell Death Percentage : %) | | | | | | | | | | |
| Concentration | 20% | 100 | 100 | 100 | 100 | 100 | 100 | 100 | 100 | 100 |
| | 10% | 100 | 100 | 100 | 100 | 100 | 89 | 91 | 88 | 100 |
| | 5% | 100 | 100 | 100 | 100 | 100 | 65 | 68 | 70 | 96 |
| | 2% | 90 | 88 | 100 | 89 | 100 | 34 | 42 | 56 | 66 |
| | 1% | 86 | 45 | 95 | 87 | 90 | 20 | 26 | 10 | 38 |
| ②Adhesiveness | | | | | | | | | | |
| | Bovine enamel | 23.3 | 22.8 | 23.8 | 17.1 | 23.4 | 18.6 | 18.9 | 21.8 | 17.2 |
| | Bovine dentin | 19.4 | 19.4 | 21.6 | 15.1 | 21.7 | 18.0 | 17.6 | 18.6 | 15.0 |
| ③Storage Stability | | | | | | | | | | |
| Discoloration | ΔL* | 0.7 | 0.8 | 0.5 | 0.7 | 0.6 | 8.3 | 7.1 | 8.3 | 6.8 |
| | Δb* | 2.0 | 2.2 | 1.8 | 2.0 | 1.9 | 10.5 | 11.3 | 19.5 | 12.6 |
| | Visual check | pale pink | pale pink | pale pink | pale blue | pale blue | dark brown | dark brown | dark green | dark green |
| Adhesiveness | Bovine dentin | 17.8 | 17.7 | 19.1 | 14.7 | 19.6 | 9.6 | 9.5 | 7.1 | 9.7 |

As is obvious from Table 4, the antibacterial compositions comprising an antibacterial salt compound, an acid group-having polymerizable monomer, a hydrophilic polymerizable monomer, distilled water and DMAEMA (Examples 14 to 18) completely killed the cells of *Streptococcus mutans* when their concentration was at least 5 %. In addition, the adhesiveness of these antibacterial compositions was good; and even after stored in a thermostat at 50°C for 1 month, the compositions still kept the color of the pigment therein, and did not discolor when observed visually, and their bonding strength to dentin lowered little. As opposed to these, however, the antibacterial compositions containing a basic compound, aromatic amine DEPT or DMPT (Comparative Examples 14 to 17) could not completely kill the cells of *Streptococcus mutans* when their concentration was 5 %. In addition, when stored in a thermostat at 50°C for 1 month, they greatly discolored and their bonding strength to dentin greatly lowered.

INDUSTRIAL APPLICABILITY

The antibacterial composition of the invention has the ability to kill cariogenic bacteria in decayed teeth or to prevent the bacteria therein from propagating and, in addition, its adhesiveness to teeth and its storage stability are both good. Therefore, the composition is effective for preventing secondary caries to be caused by the cariogenic bacteria existing in decayed teeth, and for preventing the bacteria from penetrating into the bonding interface to which it has been applied; and the composition ensures esthetic dental treatment with it.

## Claims

1. An antibacterial composition comprising (a) a polymerizable group-having antibacterial salt compound, (b) an acid group-having polymerizable monomer, (c) a hydrophilic polymerizable monomer, (d) water, and (e) a basic compound selected from alkali metal hydroxides, strong basic acid salts not having an aromatic group, and aliphatic amines.

2. The antibacterial composition as claimed in claim 1, wherein the antibacterial salt compound (a) is a polymerizable group-having, antibacterial pyridinium salt compound.

3. The antibacterial composition as claimed in claim 1 or 2, wherein the basic compound (e) is a polymerizable group-having, aliphatic tertiary amine.

4. The antibacterial composition as claimed in any of claims 1 to 3, which further contains (f) a polymerization initiator.

## Patentansprüche

1. Antibakterielle Zusammensetzung, umfassend (a) eine antibakterielle Salzverbindung mit einer polymerisierbaren Gruppe, (b) ein polymerisierbares Monomer mit einer Säuregruppe, (c) ein hydrophiles polymerisierbares Monomer, (d) Wasser und (e) eine basische Verbindung, ausgewählt aus Alkalimetallhydroxiden, stark basischen Säuresalzen, die keine aromatische Gruppe aufweisen, und aliphatischen Aminen.

2. Antibakterielle Zusammensetzung, wie in Anspruch 1 beansprucht, wobei die antibakterielle Salzverbindung (a) eine antibakterielle Pyridiniumsalzverbindung mit einer polymerisierbaren Gruppe ist.

3. Antibakterielle Zusammensetzung, wie in Anspruch 1 oder 2 beansprucht, wobei die basische Verbindung (e) ein aliphatisches tertiäres Amin mit einer polymerisierbaren Gruppe ist.

4. Antibakterielle Zusammensetzung, wie in Ansprüche 1 bis 3 beansprucht, welche weiter (f) einen Polymerisationsinitiator enthält.

## Revendications

1. Composition antibactérienne comprenant (a) un composé de sel antibactérien ayant un groupe polymérisable, (b) un monomère polymérisable ayant un groupe acide, (c) un monomère polymérisable hydrophile, (d) de l'eau et (e) un composé basique choisi parmi des hydroxydes de métaux alcalins, des sels d'acides basiques forts n'ayant pas de groupe aromatique et des amines aliphatiques.

**2.** Composition antibactérienne selon la revendication 1, dans laquelle le composé (a) de sel antibactérien est un composé de sel de pyridinium antibactérien ayant un groupe polymérisable.

**3.** Composition antibactérienne selon la revendication 1 ou 2, dans laquelle le composé basique (e) est une amine tertiaire aliphatique ayant un groupe polymérisable.

**4.** Composition antibactérienne selon l'une quelconque des revendications 1 à 3, contenant en outre (f) un initiateur de polymérisation.

Figure 1

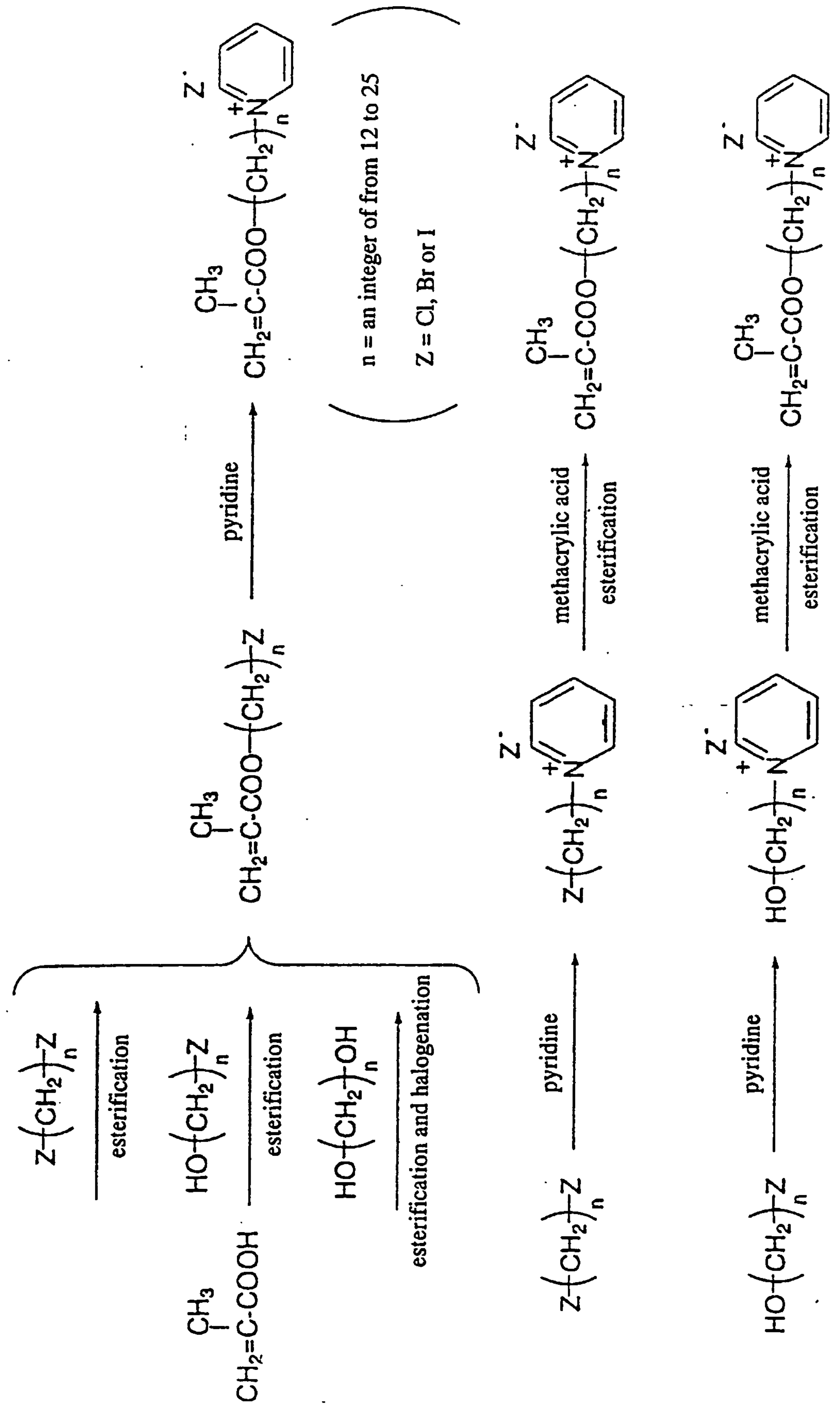
esterification
esterification
esterification and halogenation
pyridine
n = an integer of from 12 to 25
Z = Cl, Br or I
pyridine
methacrylic acid
esterification
pyridine
methacrylic acid
esterification

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 8157318 A
- WO 9951230 A
- JP 3294286 A
- JP 62281885 A
- JP 52113089 A
- JP 53067740 A
- JP 53069494 A
- JP 53144939 A
- JP 58128393 A
- JP 5192891 A
- JP 3057916 B
- JP 10245525 A